# EUROPEAN PATENT SPECIFICATION

(11) **EP 2 126 048 B1**
(45) Date of publication and mention of the grant of the patent: **20.06.2012**
(21) Application number: 08702464.2
(22) Date of filing: 18.01.2008
(51) Int. Cl.: C12N 5/00

(54) **PROCESS FOR TREATING CULTURED CELLS**
VERFAHREN ZUR BEHANDLUNG KULTIVIERTER ZELLEN
PROCÉDÉ DE TRAITEMENT DE CELLULES MISES EN CULTURE

(30) Priority: 24.01.2007 EP 07101104
(43) Date of publication of application: 02.12.2009
(73) Proprietor: Koninklijke Philips Electronics N.V., 5621 BA Eindhoven (NL)
(72) Inventor: PEETERS, Emiel, NL-5656 AE Eindhoven (NL); BROER, Dirk, Jan, NL-5656 AE Eindhoven (NL); PENTERMAN, Roel, NL-5656 AE Eindhoven (NL); KURT, Ralf, NL-5656 AE Eindhoven (NL); VAN LIEROP, Steven, NL-5656 AE Eindhoven (NL)
(74) Representative: Kroeze, Johannes Antonius
(86) International application number: PCT/IB2008/050188
(87) International publication number: WO 2008/090501

(56) References cited:
- EP-A- 0 301 777
- WO-A-95/19430
- WO-A-02/053193
- YEH ET AL: "Micromolding of shape-controlled, harvestable cell-laden hydrogels" BIOMATERIALS, ELSEVIER SCIENCE PUBLISHERS BV., BARKING, GB, vol. 27, no. 31, 1 November 2006 (2006-11-01), pages 5391-5398, XP005575037 ISSN: 0142-9612
- QIBING PEI ET AL: "Bending bilayer strips built from polyaniline for artificial electrochemical muscles" SMART MATERIALS AND STRUCTURES, IOP PUBLISHING LTD., BRISTOL, GB, vol. 2, no. 1, 1 March 1993 (1993-03-01), pages 1-6, XP002484390 ISSN: 0964-1726
- PRINZ YA V ET AL: "FREE-STANDING AND OVERGROWN INGAAS/GAAS NANOTUBES, NANOHELICS AND THEIR ARRAYS" PHYSICA E - LOW-DIMENSIONAL SYSTEMS AND NANOSTRUCTURES, ELSEVIER SCIENCE BV, NL, vol. 6, no. 1-04, 1 February 2000 (2000-02-01), pages 828-831, XP001148343 ISSN: 1386-9477

## Description

The present invention is related to a process for treating cultured cells, especially for cell handling or cell delivery in tissue engineering and cell therapy applications.

### BACKGROUND OF THE INVENTION

Regenerative medicine is a new upcoming discipline within the field of medical sciences. There are numerous methods and approaches used in regenerative medicine. They can coarsely be divided into four areas:
In tissue engineering in vitro, tissue is grown outside the body utilising scaffolds and cells. The engineered tissue is subsequently implanted in a patient in order to replace damaged or lost tissue.

In tissue engineering in vivo, scaffolds are placed in damaged tissue areas with the aim of inducing growth of cells from the surrounding healthy tissue to restore damaged tissue.

Cell therapies are based on the delivery of cells, particularly stem cells, to a damaged tissue area in order to restore the tissue function.

There are therapies utilizing growth factors, e.g. cytokines and chemokines, in order to recruit endogenous cells to the damaged tissue area. The growth factors can be delivered directly to the area of interest, e.g. via injection.

Cells play a crucial role in both in vitro tissue engineering and cell therapies where the cells are first harvested from the appropriate cell source, i.e. from the patient (autologous cell source) or from a donor (alleogenic cell source) and subsequently subjected to several different steps until they are finally introduced in the patient to replace or restore damaged tissue. Despite the tremendous progress in cell therapies and tissue engineering over the last few years, basic and essential steps, e.g. cell handling, cell growth, or ingrowth, respectively, in scaffolds, cell differentiation, cell delivery and cell retainment are still problematic and need further improvement before regenerative medicine becomes clinically relevant.

In tissue engineering *in vitro,* cells are seeded and grown in a structural matrix in the presence of bioactive molecules that promote growth and direct differentiation of the cells in order to grow new tissue in a bioreactor. The newly grown tissue is later to be implanted *in vivo.* In state of the art tissue engineering it is not possible to precisely control the 3D-distribution of different cell types in a tissue-engineered product. It is difficult to control the in-growth of cells in the 3D construct. Cell seeding onto solid porous scaffolds typically does not result in an even, homogeneous cell distribution. Underlying this problem is the fact that sufficient pore sizes are needed for cells to migrate into the interior of the scaffold and to allow sufficient diffusion of nutrients and metabolites, while on the other hand, few cells are retained in highly porous structures with large open pores and high surface-to volume-ratios are essential to facilitate population of the scaffold with large numbers of cells. It is even more problematic to control the differentiation of stems cells or progenitor cells into different cell types at different location in the 3D structural matrix. Control over the 3D distribution of different cell types is, however, essential for the construction of complex tissues such as complete organs. Furthermore, the control over the 3D distribution of different cell types is essential to provide vascularization of larger tissue engineered products.

As an alternative to the use of pre-fabricated porous scaffolds, it is known in the field to encapsulate cells in hydrogels, photo-polymerizable hydrogels and thermoreversible hydrogels. This generally results in an even, homogenous cell distribution and the highly swollen state of the gels allows sufficient diffusion of nutrients and metabolites. However, the hydrogels lack the mechanical stability needed for the engineering of skeletal tissue (e.g. bone and cartilage). Again, control over the 3D distribution of different cell types in one gel is difficult.

The basic principle of (stem-) cell therapies is to introduce (stem-) cells in an area of injury or disease. The newly introduced (stem-) cells have the ability to grow, divide and differentiate, and can repopulate the damaged site with newly formed tissue that has the same or a similar structure and function as the native tissue. The main concern in cell therapies under development lies in the fact that for many therapies large excesses of cells are needed. Often, only 1% of the injected cells are delivered and/or retained in the area of interest and survives the first week. Cells are expensive and difficult to obtain in large quantities (time consuming).

Therefore, it is undesirable that these cells are applied in large excess. More important, it is unclear what happens to the excess cells after injection. They might be harmful and form, for instance, a risk for cancer.

The problems mentioned above can be largely overcome when individual cells or pluralities of cells are encapsulated in small cell carriers or containers. It is known in the art to use microcarriers in cell cultures. The microcarriers can be solid (cell attachment only to surface) or the carriers can be porous (entrapment of cells in interior). Typical microcarriers are based on biocompatible materials such as collagen, hydroxyapatite, PGLA and bioceramics.

The use of microcarriers is generally limited to cell culturing. Only a few reports are known where microcarriers are used in tissue engineering. One recent paper reports the use of macroporous gelatine-based microcarriers seeded with osteoblast cells, in combination with a photopolymerizable material for application as an injectable scaffold , as described in Declercq H et al., Biomacromolecules 2005 (6), 1608.

Second, in WO 96/12510 epithelial cells are grown on the surface of solid microcarriers and inclusion of the carriers in hydrogels such as methyl cellulose, alginates and agarose is reported in order to obtain transplants for the treatment of burn wounds and slow healing wounds.

As a drawback, in state of the art microcarriers, the cells are attached to the surface or reside in large open pores. In effect the cells are not shielded from the environment by the microcarrier (contact between cells and environment is in fact required for cells to grow on the microcarriers).

Furthermore, present microcarriers are not responsive and therefore it is not possible to switch between exposure to the environment and protection from the environment.

Recently, a micro-machined container based on a silicon technology loaded with dedicated living cells able to produce insuline has been presented (D'Aquino, R: (2004): Good Drug Therapy: It's Not Just the Molecule - It's the Delivery, Chemical Engineering Progress 100(2), 15s). This device is loaded with cells *in vitro,* and is subsequently implanted. The device comprises a porous membrane, which allows nutrients to enter its lumen and thus feed the cells, but keeps T-cells and macrophages outside, thus preventing an immune reaction. However, fabrication of this device is very labour-intensive and therefore not suitable for cell handling and cell deliver in tissue engineering and cell therapy applications.

### SUMMARY OF THE INVENTION

It is the object of the present invention to provide microcarriers in the above meaning, in which cells can be provided, and in which the cells can be shielded from the environment for at least part of the time during application, e.g. cell handling or cell delivery.

This object is achieved by the method and the two-dimensional object as set forth under the independent claims. The dependent claims indicate preferred embodiments. In this context it is noteworthy to mention that all ranges given in the following are to be understood as that they include the values defining these ranges.

According to the invention, a process for treating cultured cells is provided, comprising the steps of
a) providing planar, two-dimensional objects ("flakes"), wherein these objects comprise a material which, upon application of an extrinsic stimulus, is transferred from the planar state into a rolled state,
b) providing cells on said planar flakes, and
c) transferring the flakes from the planar state into a rolled state by application of said extrinsic stimulus, thereby wrapping at least part of the cells.

By doing so, at least one of the following advantages can be reached for a wide range of applications within the present invention:
- By the "cell wrapping" technique it is possible to shield cells; e.g. during storage.
- The "flakes" allow to build up complex structures; as will be described later on.
- Due to the extrinsic stimulus it is possible to separate the steps of providing the cells on the flakes and the "wrapping" step, rather than being forced to grow and/or provide cells in a topological unfavourable environment.

According to a preferred embodiment, step b) comprises the substeps of:
b1) seeding cells on said planar flakes; and
b2) growing said cells.

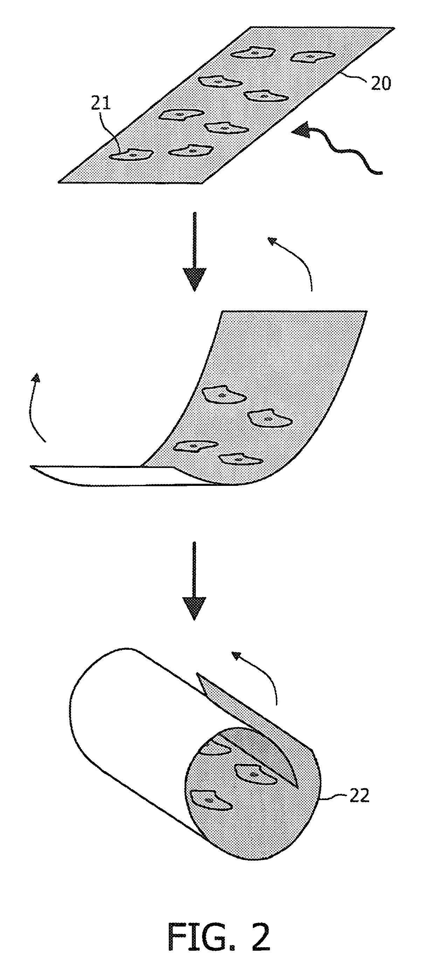

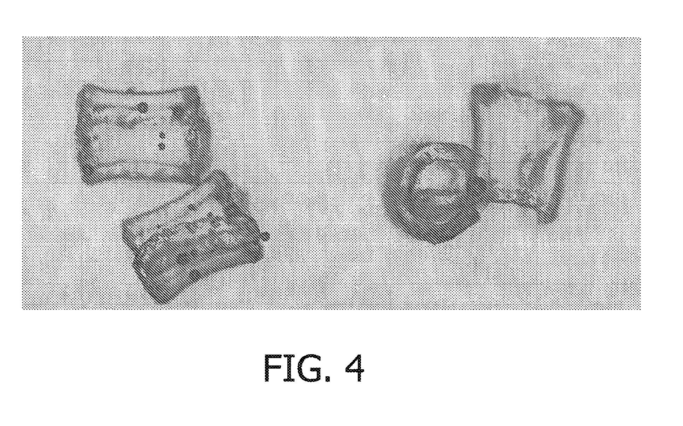

After said transfer the once planar flakes may for example adopt a cylindrical shape with open or substantially closed ends. See Figs. 2 and 4 for an example of such shape. Typical dimensions of the flakes are in the order of the dimensions of the cells or somewhat bigger than that. This means that, according to a preferred embodiment of the present invention, the size or the length of the planar hydrogel flakes is ≥ 10µm and ≤ 100mm, more preferably ≥ 10µm and ≤ 10mm, and more preferably > 20µm and ≤ 1mm, and most preferably ≥ 50µm and ≤ 500 µm.

The grown cells are wrapped inside of the rolled flakes. The said process does thus provide microcarriers for cultured cells. The rolled flakes produced with the process according to the invention will therefore be referred to as microcarriers in the following.

According to another preferred embodiment of the present invention, the thickness of the planar flakes is ≥ 100nm and ≤ 1mm, more preferably ≥ 500nm and ≤ 500µm, and most preferably ≥ 1µm and ≤ 100µm.

According to yet another an embodiment of the present invention, the inner diameter of the flakes in the rolled up state (comprising cells) is ≥ 1 µm and ≤ 5mm, more preferably ≥ 5µm and ≤ 500µm, and most preferably ≥ 10µm and ≤ 100µm.

In addition to mere cell growth, the cells can remain on the planar flakes for differentiation before the flakes are transferred into the rolled state. This means that, between steps b) and c), a differentiation step can be introduced. Likewise, cell division, cell growth, and cell profilation can be promoted between steps b) and c). The person skilled in the art may readily select from his knowledge, or from appropriate references, the conditions which are to be applied in order to achieve cell differentiation as referred to above.

Before or during transferring the flakes into the rolled state, it is preferred that they are being released from a substrate they adhere to. The release and the rolling of the flakes can, in a preferred embodiment, both be initiated by the swelling of the responsive hydrogel layer. In this embodiment, the release and transfer into the rolled state happen at the same time. In the unswollen state there are little to no stresses in the flakes, and although the adherence of the flakes to the substrate is not optimal the flakes will adhere to the substrate. In the swollen state the built-up stress and change in hydrophility of the hydrogel layer will cause release of the flakes. The cultured cells include stem cells and differentiated cells, e.g. adult mesenchymal stem cells, adult hemopoietic stem cells, adipose derived adult stem cells, embryonic stem cells, chrondrocytes, osteoblasts, osteocytes, myoblasts, cardiac myocytes, fibroblasts, B cells, T cells, dendritic cells, erythrocytes, lymphoid progenitor cells, myeloid progenitor cells, etc, of both human and non-human origin. However, for research purposes, or for the production of cells which are later being used in the production of biological matter, even immortalized cells can be used, i.e. hybridoma cells and the like.

This means as well that in case omnipotent stem cells are being used, step c) will take place before the cells start to differentiate. In case pluripotent cells are being used, a differentiation (at least in part) may take place before step c) is induced, as illustrated above.

In most cases, standard cell culture conditions will be used. Mammal cells, including human cells, for example, are preferably cultured at 37 °C and under a 5% CO₂-atmosphere in order the keep the pH in a physiological range. Standard growing media such as FCS (Fetal calf serum) may be used, and growth factors, antibiotics and the like may be added if necessary. Insect cells, plant cells and prokaryotic cells, which also fall under the scope of the present invention, will however be treated with different conditions, which are well known per se from the state of the art.

It is moreover understood that the shape of the flakes can resemble a square, a rectangle or a parallelogram. More complex shapes can however be used in order to achieve a more complex wrapped state. For example, in order to achieve a helix-like wrapped state, the flakes should have a shape which resembles an elongated parallelogram. Likewise, flakes can as well have a circular, elliptic, trapezium like, hexagonal, polygonal or triangular shape, which in each case will lead to different wrapped states. A surface structured topologically, mechanically, or in composition, and a more complex layering within the flakes can also contribute to achieve a more complex wrapped state.

In this context, it is worth mentioning that the rolled up state can include two cases, namely at least partially multilayered cylinder, as can be seen in Fig. 2, or cylindrical body just substantially closed. The latter means that there is basically no overlap of the two touching edges, which would also allow sharper angles between the closing sides of the flake.

Usually, it desired that the cells are only present on the flakes and not on the substrate in between the flakes. In order to avoid that cells settle down in the interstitium between the flakes, the surface of the substrate underneath can be modified such that cells do not adhere to it, or that the cells have a clear preference to grow on the flakes rather than on the substrate. However, in some cases it does not matter if cells also grow on the substrate. As soon as the flakes are released and rolled up, the substrate with left-over cells can be discarded.

In some special cases it can even be beneficial to have cells in the interstitium between the flakes. For instance, some difficult cell-types need co-feeder cells for growth, namely for the production of the appropriate growth factors. These feeder cells can be seeded in between the flakes, while the cells of interest are grown on the flakes.

According to a preferred embodiment of the inventive process, said stimulus is selected from the group consisting of induced change of pH, induced change of temperature, induced exposure to electromagnetic waves, induced exposure to ions, specific salts or organic compounds, or to a given concentration thereof, application of an electric field, application of a magnetic field, application of sound, application of vibrations, induced exposure to, or an induced suppression of, enzymes and other biomolecules, and/or induced exposure to a solvent composition.

As regards a thermal stimulus, it is for example preferable that upon heating (e.g. ≥ 36 °C) the flakes are in the planar state, whereas they are transferred into a rolled state upon cooling (e.g. ≤ 35 °C). This is especially beneficial, as by cooling the flakes (and the cells comprised therein) cells will stop growing, and their metabolic rate is reduced. Cells can thus be stored for transport or prepared for the respective application, without injury or detrimental effects. It is as well preferred that the stimuli applied are selected as not to deteriorate the physiology of the cells.

Moreover, in a preferred embodiment it is provided that the flakes comprise a stimulus responsive material which has reversible swelling properties, or the like, i.e. the said stimulus can be applied many times.

It is understood that the term "electromagnetic waves" includes visible light, ultraviolet and infrared light, X-ray, microwaves, radiowaves and the like. It is further understood that the term "sound" includes ultra- and infrasound, as well as audible sound.

In a further preferred embodiment the flakes comprise labels or markers. Such labelling can for example be accomplished by use of dyes, magnetic beads, X-Ray markers, MRI-markers or targeting moieties like antigens, lectins, reactive groups, and the like.

It is a much better way to label the flakes than to label the cells themselves, as such labelling may detrimentally affect cell organelles including the nucleus and the nucleic acids, as well as cell physiology, cell enzymes, cell metabolism and the like.

Such labelling does for example allow the design of tissues consisting of more than cell type, as the respective flakes can be recognized, selected and positioned in a scaffold, for example, by means of their labelling. Such positioning can also be done automatically, e.g. by a dedicated robot, in which case the respective labels or markers (e.g. fluorescent markers) are detected automatically by the robot.

The labelling does moreover allow the cell-type specific application of growth factors, which is very helpful in the engineering of tissues which comprise different cell types. Again, a pipetting robot may be used for this purpose.

When being used in cell therapy, the layer that is not contacting the cells may be labelled with tissue specific antigens. Thus, the delivery of the flakes and their content to the target site is supported.

In this context, X-ray markers and MRI-markers can also facilitate the targeting of the rolled flakes. For this purpose, an X-ray-tomograph or an MRI-tomograph can be used. Moreover, the respective labels could be used to control the wrapping and unwrapping of the flakes inside the target organism, or their integrity or degradation, respectively.

This approach helps to reduce the number of cells needed in cell therapy, for example, which results in a reduction of costs and resources, as cultured cells are expensive, and their production is time- and labour intensive. More important, the number of cells which do not remain in the area of therapy is reduced. This again reduces any unwanted side effects of cells which float freely in the target body, and may thus cause cancer or other diseases.

When multiple micro-containers according to the above definition are used for tissue engineering, reactive groups as described above can be used to attach the microcarriers to each other and thus form a mechanically stable cell-loaded scaffold.

Such reactive groups can for example form covalent bonds or ion bonds between different microcarriers. However, other moieties fall under the definition "reactive groups", as referred to herein, as well. Such moieties are, for example, pairs of antigens and their corresponding antibodies, both linked to microcarriers, lectins, streptavidin/biotin and the like.

Furthermore, it is preferred that the flakes comprise agents which enhance the biocompatibility. For example, the layer that is not contacting the cells may comprise an anticoagulat, e.g. heparin moieties, to avoid blood clotting. The person skilled in the art will select other agents which enhance the biocompatibility according to the specific needs. For example, it is possible to modify the layer contacting the cells with specific anchoring molecules, growth factors and the like.

In yet another preferred embodiment the flakes comprise a material consisting of a hydrogel.

The term "hydrogel" as used herein implies that at least a part of the respective material comprises polymers that in water form a water-swollen network and/or a network of polymer chains that are water-soluble. Preferably the hydrogel permeation layer comprises in swollen state ≥ 50% water and/or solvent, more preferably ≥ 70% and most preferred ≥ 80%, whereby preferred solvents include organic solvents, preferably organic polar solvents and most preferred alkanols such as Ethanol, Methanol and/or (Iso-) Propanol.

A responsive hydrogel is particularly preferred. In the sense of the present invention, the term "responsive" means and/or includes especially that the hydrogel is responsive in such a way that it undergoes a change of shape and total volume upon a change of a specific parameter, or the application of a specific stimulus, the nature of which is further specified above (e.g. induced change of pH, induced change of temperature, induced exposure to electromagnetic waves, induced exposure to specific salts or organic compounds, or to a given concentration thereof, application of an electric field, application of a magnetic field, application of sound, application of vibrations), Other stimuli include the presence or concentration of dedicated analytes such as enzymes or other biomolecules. (see comment above).

Hydrogels are known to be shape sensitive to pH, ion concentration, temperature, solvent composition and electric potential. These parameters may cause a change in phase, shape, mechanics, refractive index, recognition or permeation rates that subsequently can be reversed to return the material to its original state. Stimuli-sensitive hydrogels have also been integrated with enzymes, protein mimics, and antibodies to design controllable actuators. These hydrogels have been shown to swell on addition of a target molecule. The amount of swelling of these hydrogels was attributed to changes in non-covalent interactions within the polymer network. The hydrogels can also be designed to swell upon presence of a target molecule; even they can be constructed in a way that the magnitude of swelling can be proportional to the concentration of ligand present.

According to an embodiment of the present invention, the hydrogel material comprises a material selected out of the group comprising poly(meth)acrylic materials, substituted vinyl materials or mixtures thereof, as well as include epoxydes, oxetanes, and thiolenes.

According to another embodiment of the present invention, the hydrogel material comprises a poly(meth)acrylic material made out of the polymerization of at least one (meth)acrylic monomer and at least one polyfunctional (meth)acrylic monomer.

According to yet another embodiment of the present invention, the (meth)acrylic monomer is chosen out of the group comprising (meth)acrylamide, hydroxyethyl(meth)acrylate, ethoxyethoxyethyl(meth)acrylate or mixtures thereof.

According to still another embodiment of the present invention, the polyfunctional (meth)acrylic monomer is a bis-(meth)acryl and/or a tri-(meth)acryl and/or a tetra-(meth)acryl and/or a penta-(meth)acryl monomer.

According to an embodiment of the present invention, the polyfunctional (meth)acrylic monomer is chosen out of the group comprising bis(meth)acrylamide, diethyleneglycoldi(meth)acrylate, triethyleneglycoldi(meth)acrylate, tertraethyleneglycoldi(meth)acrylatetripropyleneglycol di(meth)acrylates, pentaerythritol tri(meth)acrylate polyethyleneglycoldi(meth)acrylate, ethoxylated bisphenol-A-di(meth)acrylate , hexanedioldi(meth)acrylate or mixtures thereof.

Other materials which turned out in tests carried out by the inventors to be suitable for the above purposes include Ethylhexyl acrylate, Hydroxyethyl methacrylate, PNIPAA-co-isobutylmethacrylate (80:20), PMMA, PMMA-co-trimethylolpropane triacrylate, TMPTA, DEGDA, DEGDMA, Polystyrene, PMMA-co-DEGDA (2:1), PMMA-co-DEGDMA (2:1), PS-co-TMPTA (2:1), PS-co-DEGDA (2:1), PS-co-DEGDMA (2: 1) and tris 2-hydroxyethyl isocyanurate triacrylate.

According to an embodiment of the present invention, the hydrogel material comprises an anionic poly(meth)acrylic material, preferably selected out of the group comprising (meth)acrylic acids, arylsulfonic acids, especially styrenesulfonic acid, itaconic acid, crotonic acid, sulfonamides or mixtures thereof, and/or a cationic poly(meth)acrylic material, preferably selected out of the group comprising vinyl pyridine, vinyl imidazole, aminoethyl (meth)acrylates or mixtures thereof, co-polymerized with at least one monomer selected out of the group neutral monomers, preferably selected out of the group vinyl acetate, hydroxyethyl (meth)acrylate (meth)acrylamide, ethoxyethoxyethyl(meth)acrylate or mixture thereof, or mixtures thereof.

It is known for a wide range of these co-polymers to change their shape as a function of pH and to respond to an applied electrical field and/or current. Therefore these materials may be of use for a wide range of applications within the present invention.

According to an embodiment of the present invention, the hydrogel material comprises a substituted vinyl material, preferably vinylcaprolactam and/or substituted vinylcaprolactam.

According to an embodiment of the present invention, the hydrogel material is based on thermo-responsive monomers selected out of the group comprising N-isopropylamide, diethylacrylamide, carboxyisopropylacrylamide, hydroxymethylpropylmethacrylamide, acryloylalkylpiperazine and copolymers thereof with monomers selected out of the group hydrophilic monomers, comprising hydroxyethyl(meth)acrylate, (meth)acrylic acid, acrylamide, polyethyleneglycol(meth)acrylate or mixtures thereof, and/or co-polymerized with monomers selected out of the group hydrophobic monomers, comprising (iso)butyl(meth)acrylate, methylmethacrylate, isobornyl(meth)acrylate or mixtures thereof. These co-polymers are known to be thermo-responsive and therefore may be of use for a wide range of applications within the present invention.

A preferred example for these responsive hydrogels is Poly-n-isopropylacrylamide (PNIPAA).

The above mentioned hydrogels are highly permeable for low-molecular compounds, i.e. salts, sugars, lipids, growth factors, oxygen, and the like. For this reason, flakes comprising these gels will provide appropriate growing conditions for cells, even in the wrapped state.

In another embodiment, the layer which is in contact with the cell is not an hydrogel material. In these cases, it is preferred that the contact layer is structured such that it contains holes or micropores to allow the transport of nutrients and metabolites. However, transport can also take place trough the "open sides" of the wrap.

Furthermore, it is preferred that the flakes comprise a bilayer structure. By selecting suitable materials for the different layers, a different swelling of the two layers can be accomplished upon a given stimulus, e.g. due to different thermal expansion coefficients, water uptake or the like. This difference provides the driving force needed for the movement, i.e. the curling, of the flakes, which results in the rolled state. In a preferred embodiment of the former, the flakes consist of a non-responsive layer and a layer made from a responsive hydrogel.

Flakes can also comprise a tri- or multilayer structure or a gradient structure, i.e. a vertical concentration gradient of swelling or gelling components, which may result in similar behaviour. The above mentioned gradient can be produced with methods well know for the person skilled in the art. A gradient mixer, as commonly used to produce gradient gels for electrophoresis, can for example be used for this purpose. Another approach to create composition gradients is to induce the latter by use of a vertical gradient in polymerisation rate (e.g. intensity gradient by using a UV absorber) .

In this context, it is worth mentioning that at least one of the above mentioned layers can comprise a structured surface.

A tri-layered embodiment can for example comprise a top layer which is very thin, thus not affecting the stress mechanics which lead to the rolling movement, but which comprises a composition that is well compatible with, or even promotes, cell growth.

Yet, in cases where adherent cell sheets with high confluency are grown on the flakes it is as well possible to use single layer flakes, i.e. flakes without a gradient or a stratified arrangement. In these cases the cell sheet itself acts as the second layer and the differences in expansion behaviour between the single layered flakes and the adherent cell sheet results in the rolling-up of the flakes into the wrapped state. This feature is especially useful for another preferred embodiment of the present invention, wherein the adhesion of cells is used to determine in which cell cycle state the cells are, as in some states a cell applies more tensile forces to its adhesion points than in other states.

In yet another preferred embodiment it is provided that the flakes comprise a structured cell contacting surface. This may stimulate cell adhesion, or help to specifically direct the growth and orientation of the cells. In these cases, a structured cell contact layer or a structured non contacting layer can induce a preferred rolling direction of the flake.

It is moreover preferred that during cell culturing, external stimuli are being applied in order to influence cell growth or cell differentiation.

Such stimuli can for example be selected from the group consisting of the application of growth factors, application of mechanical stress, or the application of electric or magnetic fields. In particular, these stimuli can enforce or prevent cell differentiation, according to cell types and to the respective application, as well as the direction and shape of cell growth can be controlled.

Growth factors can also be added to the layer that is in contact with the cell in order to provide that cell growth is still stimulated once the cells have been wrapped.

Furthermore, it is preferred that the flakes are disposed or produced on a support structure before cells are seeded.

In a preferred embodiment the flakes comprise a material which is biodegradable and/or biologically safe. This is an important feature both for tissue engineering and for use of the flakes in cell therapy. For example, the use of biodegradable material allows, under certain circumstances, that the flakes are directly administered to a subject. Likewise, said material, when the flakes are being used for *in vitro* tissue engineering, can be selected as to slowly disintegrate, wherein the speed of disintegration corresponds to the speed of cell growth and of the production of extra cellular matrix by the growing cells. In this embodiment, the extracellular gel matrix is stepwise replaced by living cell matter.

Suitable biodegradable materials are for example described by Gunatillake P and Adhikari R, Eunopean Cells and Materials (5) 2003, 1-16. Among these are Poly(glycolic acid) (PGA), poly(lactic acid) (PLA) and their copolymers and derivatives, like Poly(d,l-lactic-co-glycolic acid), Polylactones like Poly(caprolactone) (PCL) and their derivatives, Poly(propylene fumarate) (PPF) and its derivatives, Polyanhydrides like Poly [1,6-bis(carboxyphenoxy)hexane], Tyrosine-derived polycarbonates and their derivatives, Polyorthoesters (POE) and their derivatives, Polyurethanes (PU) with non-toxic degradation products like lysine diisocyanate (LDI, 2,6-diisocyanatohexanoate) and other aliphatic diisocyanates like hexamethylene diisocyanate (HDI) and 1,4-butanediisocyanate, e.g. Poly(glycolide-co-γ-caprolactone), Polyphosphazenes like Ethylglycinate Polyphosphazene and their derivatives, and so forth.

Other biodegradable polymers comprise poly(maleic acid), poly(p-dioxanone), poly(trimethylen-carbonate), poly(3-hydroxibutarate), poly(3-hydroxyvalorate and their copolymers. A class of suitable responsive biodegradeable polymers is Poly(N-(2-hydroxypropyl) methacrylamide mono/dilactate), as described by Soga O et al, Biomacromolecules 2004 (5) 818-821.

Other suitable biodegradable materials comprise alginate, hyaluronic acid, chitosan, collagen, gelatin, silk or combinations thereof.

These biodegradable materials can be used by themselves, or they are being used in a network together with crosslinking agents. The created network will disintegrate after some time, and, given the crosslinking agents have small molecular weights, the latter will be washed out. In another embodiment, the biodegradable material is immobilized in a network that consists of non-biodegradable matter.

In another preferred embodiment it is provided that the flakes comprise a material which disintegrates upon application of an extrinsic stimulus. This feature is for example useful in cell therapy, in order to enhance the release of the cells at the target site. This can for example be accomplished by application of ultrasound or infrared light, as both exhibit good penetration into the human or animal body.

For this purpose, the rolled flakes are injected and targeted via targeting moieties on the outside the rolled flakes, an the latter can un-roll upon application of an external stimulus and thus exposing the cells to the area of interest. Alternatively, the flakes are not targeted but homogeneously distributed over the body, while the external stimulus is focussed only on the area of interest. Only there the rolled flakes will unroll and release their cellular content.

Preferably, the cells used in the procedure according to the invention are adhering cells. However, suspended cells may also be used. In these cases, it can be provided that the liquid comprising the suspended cells remains in the rolled flakes due to capillary forces.

A preferred application of the flakes produced with the above process comprises that after transferring the flakes from the planar state into the rolled state, the rolled flakes are being distributed in a predefined spatial pattern in order to produce a three dimensional tissue. This approach may likewise be used for tissue engineering *in vitro* and *in vivo.* In order to achieve this aim, a scaffold may be used which predetermines the shape of the tissue, or organ, respectively, that is to be produced, and in which the microcarriers according to the invention are deposited.

With this approach it is possible to obtain a heterogeneous distribution of different cell types which have been cultured and specifically treated in different cell cultures and subsequently wrapped in different flakes). In this way it is possible to control precisely which cell type is at which place in the newly grown tissue. Alternatively, one single omni- or pluripotent cell type (stem cell or progenitor cell) can be cultured on different flakes containing different factors stimulating the differentiation into different cell types. The wraps can then be distributed in a predefined matter and the cells will mature into different tissue types during tissue engineering. Thus, a bone-cartilage interface construct can for example be obtained.

Likewise, it is possible to assemble a plurality of microcarriers by means of reactive groups as set forth above in a three dimensional way, thus producing a tissue or organ, respectively, without use of a dedicated scaffold. This again is preferably done by means of a robot.

Another preferred application of the flakes provides that after transferring the flakes from the planar state into the rolled state, the rolled flakes are being aligned with their open ends to one another. In this way, vessels or continuous neurons can be formed. Such alignment of the rolled flakes can be stimulated by external factors such as geometrical restraints, external magnetic or electric fields, flow and shear, and the like.

The same type of vessel-like tissues can however be achieved when flakes are being used which, are transfer, adopt a helix like shape, as set forth above.

Still another preferred application of the flakes provides that different flakes bearing grown cells are superimposed before transferring the flakes from the planar state into the rolled state. By transferring the superimposed flakes into the rolled state, one achieves a coaxial arrangement of cells, which may have different origin. This enables the production of tubular tissues, like blood vessels, with a stratified arrangement of different cells, like endothelium, connective tissue, vascular smooth muscle cells and the adventitia (containing nerves).

The same result can however be achieved by depositing and aligning rolled-up flakes onto substrates with patterned planar flakes bearing cells. Upon application of the above mentioned stimulus, these flakes will transfer from the planar state into the rolled state flakes, thus wrapping around the deposited, rolled-up flakes. Again, the same type of vessel-like tissues can be achieved when flakes are being used which, are transfer, adopt a helix like shape, as set forth above.

Another preferred application of the flakes provides that the rolled flakes are deep-frozen. By this means, they can be stored for future use, the latter being specified elsewhere in this specification.

Another preferred application of the flakes provides that the rolled flakes are delivered to a damaged tissue area. In this embodiment, stem cells, which help restore the tissue function of the damaged tissue, are preferably used. Such approach is very promising for the treatment of neurodegenerative diseases, like Alzheimer's or Parkinsson's desease, as well as for the repair of necrotic tissues, as results from cardiac stroke, for example.

Here again, the microcarriers according to the invention provide a means for targeting the cells directly to the site of damage. This results in a reduction of costs and resources, and, moreover, the number of cells which do not remain in the area of therapy, and which eventually may cause cancer or the like, is reduced.

Furthermore, another preferred embodiment provides that the flakes comprising cells are administered orally to a subject, i.e. they are swallowed. According to the composition of the flakes, which can be selected to be resistant to saliva, gastric fluids and enzymes and/or to the enzymes of the small intestine, the pancreas or the gallbladder. By this means, it can be accomplished that the flakes remain intact in order to protect the cells until they reach predefined locations in the intestines. There the flakes could unroll, or disintegrate, respectively, due to the local pH or the presence of particular agents, e.g. enzymes, and let the cells do their work.

Still another preferred embodiment comprises that the rolled flakes are being implanted in a subject, either by injection or surgically, in order to take over endocrinic functions. In this case, the flakes contain endocrinic cells, i.e. pancreas cells, or cells producing endocrinic agents, like insulin. Targeting of the flakes is possible, as set forth above.

Due to the permeability of the flakes nutrients enter the lumen and thus feed the cells. Likewise, endocrinic agents can be secreted. At the same time, T-cells and macrophages are kept outside, thus preventing an immune reaction.

According to another aspect of the invention, a process for the manufacture of a two-dimensional object ("flake") according to any of the aforementioned claims is provided, comprising the steps of:
a) providing a mould comprising a grid which creates wells defining the shape of the flakes;
b) casting a precursor material into the mould; and
c) curing the cast material to obtain flakes.

It is preferred that after curing the flakes are released from the mould and then used according to the above described processes. However, flakes can also remain in the mould. In this embodiment, the mould may for example serve as a support structure for subsequent cell culture.

It can be provided that this process is used to provide bi-, tri- or multilayered flakes. In this case, the casting and curing steps are to be repeated. By using different precursor materials for the different layers, it can be achieved that, as set forth above, the resulting flakes are transferable from a planar state into a rolled state by application of an extrinsic stimulus. Suitable materials are described in detail above. The curing can for example be accomplished by application of UV light, electron rays, heat or dedicated curing agents. However, self curing material can also be used.

In a preferred embodiment of this process it is provided that several layers are cast into the mould in order to obtain multi-layered flakes. Likewise, it may be provided that throughout casting, a gradient structure is accomplished in the flakes, e.g. a vertical concentration gradient of swelling or gelling components.

In a preferred embodiment of this process it is provided that several layers are cast into the mould in order to obtain multi-layered flakes. This means that a first layer is first cast and cured. Thereafter a second layer is cast and cured , and so on. Likewise, it may be provided that throughout casting, a gradient structure is accomplished in the flakes, e.g. a vertical concentration gradient of swelling or gelling components. The gradient can for example be formed by applying a gradient in light intensity over the layer during curing.

Furthermore, it may be provided that any of the following additives is added to the precursor material:
a) agents which enhance the biocompatibility;
b) biodegradable and/or biologically safe material;
c) labels or markers; and/or
d) growth factors;
e) antibiotics.

In yet another preferred embodiment, it is provided that before or after curing a structured surface is accomplished in the flakes. This may for example be done by using a structured template which is pressed on the surface of the flakes. It is also possible to photopolymerize said hydrogel layer, e.g. with an absorber to get a gradient in thickness direction. In this context, a double (or multiple) UV exposure can for example be used. Preferably, one of the steps can comprise the use a mask in order to achieve a surface pattern.

Another option is to use polarized light in order to create a structured surface. In this case, a linearized structure can by obtained by selective curing, or photolysation, in the plane of polarization. By repeating this process with different planes of polarization, a grid pattern or the like can be created on the surface.

According to another aspect of the invention, a two-dimensional object ("flake") is provided, having structural and/or material properties as set forth in the above process, or made with the above manufacturing process.

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

In another embodiment of the present invention, a process for the manufacture of a two-dimensional object ("flake") according to any of the aforementioned claims is provided, which comprises the following steps:
a) providing a planar substrate,
b) coating said substrate with a precursor material,
c) curing the coating material with help of a patterned light source, and
d) rinsing the non-cured precursor material.

This is an alternative process wherein the shape of the flakes is obtained by means of photolithography. This means that the shape of the flakes is determined by the pattern, as referred to above, of the light source, which is, in a preferred embodiment, a UV source. The pattern can for example be obtained by a pattern mask, or by a scanning laser.

Again, it can be provided that this process is used to provide bi-, tri- or multilayered flakes. In this case, the coating and curing steps are to be repeated. By using different precursor materials for the different layers, it can be achieved that, as set forth above, the resulting flakes are transferable from a planar state into a rolled state by application of an extrinsic stimulus.

An example which falls under the definition of the above process is given in the examples.

### BRIEF DESCRIPTION OF THE DRAWINGS

Additional details, features, characteristics and advantages of the object of the invention are disclosed in the subclaims, the figures and the following description of the respective figure and examples, which, in an exemplary fashion, show preferred embodiments of a process and flakes according to the invention.
Fig. 1 shows the first steps of a process according to the invention
Fig. 2 shows the succeeding steps of a process according to the invention
Fig. 3 shows different uses for the thus produced microcarriers
Fig. 4 shows a photomicrograph of two microcarriers according to the invention, comprising a bilayer structure containing a responsive hydrogel material

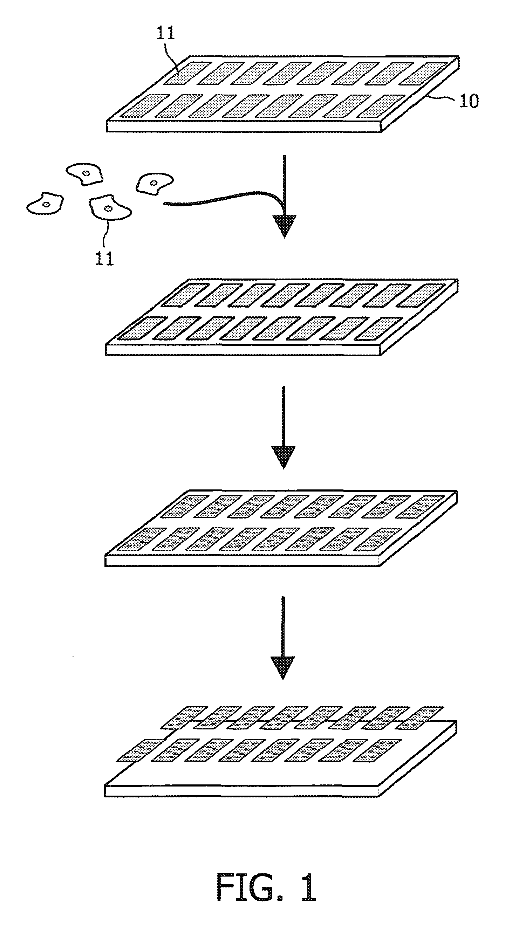

Fig. 1 shows the first steps of a process according to the invention. Therein, a support support structure 10 is provided upon which flakes 11 are being disposed or produced. Cells are then seeded on the flakes, and the substrate is kept under conditions that enable cell growth. Before the flakes are further processed, they are released from the support structure. Typical dimensions of the flakes are in the order of the dimensions of the cells or somewhat bigger than that, e.g. 100 x 100 µm.

It is obvious from Fig. 1 that the cells are only present on the flakes and not on the substrate in between the flakes. In order to avoid that cells settle down in the interstitium between the flakes, the surface of the substrate underneath can be modified such that cells do not adhere to it, or that the cells have a clear preference to grow on the flakes rather than on the substrate. However, in some cases it does not matter if cells also grow on the substrate. As soon as the flakes are released and rolled up, the substrate with left-over cells can be discarded.

In some special cases not shown here it can even be beneficial to have cells in the interstitium between the flakes. For instance, some difficult cell-types need co-feeder cells for growth, namely for the production of the appropriate growth factors. These feeder cells can be seeded in between the flakes, while the cells of interest are grown on the flakes.

Fig. 2 shows the succeeding steps of a process according to the invention. A flake 20 with cells 21 is exposed to an extrinsic stimulus, symbolized by the undulated arrow.

Said stimulus may consist of heat, a pH-change, or exposure to electromagnetic waves, for example (see specification for further details). The exposure to said stimulus elicits a response in the flakes, namely that the former are being transferred from a planar state into a rolled state, as indicated by the respective arrows. In this manner, microcarriers as defined above are being produced.

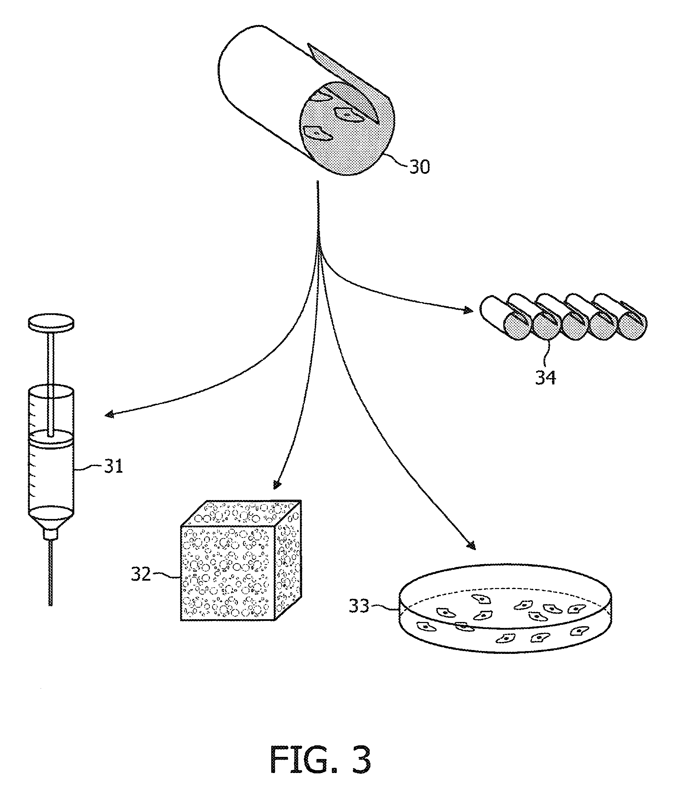

Fig. 3 shows different uses for the thus produced microcarriers. For example, the microcarriers can be used for cell therapy. For this purpose, the microcarriers can be administered to a patient by injection, as symbolized by a syringe 31. Furthermore, the microcarriers can be disposed in a scaffold 32 as it is being used for *in vitro* tissue engineering. The microcarriers can however be used for storing cells, e.g. by freezing, which are then being used for conventional cell culture, as symbolized by a petri dish 33. Last but not least, the microcarriers can by themselves be used for 3-dimensional tissue engineering.

For this purpose, several microcarriers can for example be coupled to one another with help of reactive groups, which build up ion bonds or covalent bonds, or with antigen/antibody moieties, straptividin/biotin and the like, to build up a three-dimensional network 34.

Fig. 4 shows a photomicrograph of two microcarriers according to the invention, comprising a bilayer structure containing a responsive hydrogel material. The microcarriers are 700 x 1400 µm in size, and rolled up they have a diameter of approximately 500 µm. The stimulus used to transfer the flakes from the planar state to the wrapped state was a thermal stimulus. To induce the wrapping movement, the flakes were placed in a water bath at room temperature. At room temperature the responsive hydrogel shows strong swelling in water and as a result the patterned flakes were released from the substrate and rolled. Upon heating the water bath above 33°C, the hydrogel collapses (PNIPAA has a lower critical solution temperature (LCST) at 33°C) and the hydrogel layer shrinks. As a result, the bi-layers unroll upon heating the water bath above 33°C.

### EXAMPLES

In the following, the present invention is demonstrated by means of examples, which by no means should be understood as to limit the scope of the invention.

### 1. PRODUCTION OF RESPONSIVE BILAYER FLAKES

### 1.1 non-responsive top layer comprising Ebecryl

To demonstrate the production of bi-layer flakes for the purpose of wrapping cells, Ebecryl 1810 (which is being used as non-responsive top layer), supplemented with 0.1 wt% initiator (Irgacure 65 1) and 1 : 10 initiator : inhibitor (4-methoxyphenol), was spin coated on a glass substrate (treated with 10 minutes UV-ozone) at 3000 RPM for 30 seconds. Subsequently, the substrate was positioned beneath two masks, one with rectangles (dim. 700 µm x 1400 µm) to define the flakes and one striped to define a striped Ebecryl pattern within each flake, and irradiated for 1 second in the UV-setup UV-2 with filter. Thereafter, the products are rinsed with isopropyl alcohol.

Subsequently, the glass plate with patterned Ebecryl layer is used as one of two substrates to make a cell as formed by two plan parallel substrates. The patterned Ebecryl layer is positioned on the inside of the cell. The spacing of the cell is Set to be 50 micron by using 50 micron thick tape as spacers and the cell is filled via capillary forces with a reactive mixture consisting of 50 wt% n-isopropylacrylamide (NIPAA) (1 mol% di-ethyleneglycoldiacrylate (DEGDA)) in 50/50 wt% water/methanol. The cell is placed beneath a shadow mask (squared features) and irradiated with UV -light for 4 minutes. After irradiating the cell was opened by removing the top substrate and rinsed with water. Then the bottom substrate, containing the polymerized composite elements forming the flakes, was placed in a water bath at room temperature. At room temperature the PNIPAA hydrogel shows strong swelling in water and as a result the patterned flakes were released from the substrate and rolled up in the direction perpendicular to the Ebecryl 18 10 stripes. Upon heating the water bath above 33°C the PNIPAA hydrogel collapses (PNIPAA has a lower critical solution temperature (LCST) at 33°C) and the hydrogel layer shrinks. As a result, the bi-layers unrolled upon heating the water bath above 33°C.

### 1.2. poly(methylmethacrylate) made by MMA polymerization as non-responsive top layer

To demonstrate the production of bi-layer flakes for the purpose of wrapping cells,
methylmethacrylate, supplemented with 3 wt% crosslinker (DEGDMA), 2 wt% initiator (Irgacure 651) and 3:1 initiator: inhibitor (4-methoxyphenol), was spincoated on a glass substrate (treated with 10 minutes UV-ozone) at 1000 RPM for 30 seconds. Subsequently the substrate irradiated for 100 seconds in the UV setup through a set of two masks (to obtain the same pattern as described in example 1), placed in stainless steel container with a UV transparent glass lid under nitrogen atmosphere). Subsequently, the products are rinsed with isopropyl alcohol. Further steps are similar to the procedures of creating bilayers with ebecryl 1810 as non-responsive layer.

### 1.3. poly(methylmethacrylate) made by PMMA entanglement as non-responsive top layer

To demonstrate the production of bi-layer flakes for the purpose of wrapping cells, a cell consisting of one adherent side (A174 silane) and one fluorosilane coated side and 50 micron thick tape as spacers is capillary filled with a reactive mixture consisting of 50 wt% n-isopropylacrylamide (NIPAA) (1 mol% di-ethyleneglycoldiacrylate (DEGDA)) in 50/50 wt% water/methanol. The cell is placed beneath a shadow mask (squared features) and irradiated with UV -light for 3 minutes. After irradiating the cell was opened by removing the top substrate and rinsed with water. Then placed on the hotplate at 90°C for 2 minutes, placed beneath the photomask in the UV setup and irradiated for 120 seconds. Finally the substrate is washed in anisole (3 minutes) and individual flakes were obtained by releasing them from the substrate keeping the substrate in 10% HF for 5 minutes.

### 1.4. single UV exposure

To demonstrate the production of bi-layer flakes for the purpose of wrapping cells, 50 wt% PMMA (70:30 trimethylolpropane triacrylate), supplemented with 1 wt% photo initiator (Irgacure 651) in anisole (or toluene) is spincoated on the a glass substrate at 3000 RPM for 30 seconds. The coated substrate is kept at 80 deg. for 1 minute. Then, a cell is made by coupling this substrate to a second substrate, while keeping them separated at 50 micron distance by scotch tape as spacers. Subsequently, the cell is capillary filled with a reactive mixture consisting of 60 wt% n-isopropylacrylamide (NIPAA) (1 mol% di-ethyleneglycoldiacrylate (DEGDA)) in 50/50 wt% water/methanol + 1% Irgacure 651. The cell is placed beneath a shadow mask (squared features) and irradiated with UV -light for 90 seconds. Finally, the cell is opened and the substrate containing the flakes is washed in anisole (3 minutes).The individual flakes were obtained by releasing them from the substrate by swelling in water.

### 2. CELL-WRAPPING IN CONSTRUCTED BI-LAYER FLAKES

In order to demonstrate wrapping of eukaryotic cells, substrates were prepared containing a pattern of bilayers as prepared according to method 3. Typical dimensions of the patterned flakes are in the order of the dimensions of the cells or somewhat bigger than that, 700 x 1400 µm.

The lower layer, attached to the substrate, is the PNIPAA layer. On top of the PNIPAA layer PMMA was applied to provide a good cell adhesion. The flakes are maintained at 37 °C. At this temperature the PNIPAA is in the collapsed state and there are no (or almost no) stresses between the top and the bottom layer, and although the adhesion between the substrate glass and the PNIPAA is relatively low, the patterned flakes keep their flat shape and maintain adhered to the glass substrate. Carefully keeping the composite of glass and bilayer flakes at 37 °C they are placed in a Nunclon Asurface Easyflask. A-431 human carcinoma epidermoid cells were seeded onto the substrate and supplemented with the appropriate medium D-MEM (Dulbecco's Modified Eagle Medium) supplemented with 10% FCS (Fetal Calf Serum), 1% of PenStrep (Penicillin/Streptomycin) and 1 % GlutaMAX medium, at 37 °C in humidified atmosphere with 5% CO₂. Cells are grown at 37°C in an incubator in humidified atmosphere with 5% CO₂.

After sufficient confluency of the cells has been reached and the cells are adhered to the PMMA top layer the temperature is brought to room temperature. By this operation the PNIPAA hydrogel layer swells and the flakes roll up losing their adhesion to the glass substrates and the cells are automatically wrapped to a small microcarrier.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art Accordingly, the foregoing description is by way of example only and is not intended as limiting. The invention's scope is defined in the following claims Furthermore, reference signs used in the description and claims do not limit the scope of the invention as claimed.

## Claims

1. A process for treating cultured cells, comprising the steps of
a) providing planar, two-dimensional objects (flakes), wherein these objects comprise a material which, upon application of an extrinsic stimulus, is transferred from the planar state into a rolled state,
b) providing cells on said flakes, and
c) transferring the flakes from the planar state into a rolled state by application of said extrinsic stimulus.

2. The process according to claim 1, whereby step b) comprises the substeps of
b1) seeding cells on said flakes, and
b2) growing said cells.

3. The process according to any of the aforementioned claims, whereby said stimulus is selected from the group consisting of induced change of pH, induced change of temperature, induced exposure to electromagnetic waves, induced exposure to specific salts or organic compounds, or to a given concentration thereof, application of an electric field, application of a magnetic field, application of sound, application of vibrations.

4. The process according to any of the aforementioned claims, whereby the flakes comprise a material consisting of a hydrogel.

5. The process according to any of the aforementioned claims, whereby the flakes comprise a bilayer structure, a trilayer structure, a multilayer structure and/or a gradient structure.

6. The process according to any of the aforementioned claims, whereby after transferring the flakes from the planar state into the rolled state, the rolled flakes are being distributed in a predefined spatial pattern in order to produce a three-dimensional tissue.

7. The process according to any of the aforementioned claims, whereby after transferring the flakes from the planar state into the rolled state, rolled flakes comprising different cell types are being distributed in a predefined spatial pattern in order to produce a three-dimensional, heterogeneous tissue.

8. The process according to any of the aforementioned claims, whereby after transferring the flakes from the planar state into the rolled state, the rolled flakes are being aligned with their open ends to one another.

9. The process according to any of the aforementioned claims, whereby different flakes bearing grown cells are superimposed before transferring the flakes from the planar state into the rolled state.

10. The process according to any of the aforementioned claims, whereby after transferring the flakes from the planar state into the rolled state, the rolled flakes are delivered to a damaged tissue area.

11. A process according to any of claims 4 to 10 for the manufacture of a two-dimensional object (flake) as microcarrier for cells, comprising the steps of:
a) providing a mould comprising a grid which creates wells defining the shape of the flakes;
b) casting a precursor material into the mould; and
c) curing the cast material to obtain flakes.

12. A two-dimensional object (flake) as microcarrier for cells, the object being transferable into a rolled state by application of an extrinsic stimulus forming a cylinder or cylindrical body and made with a process according to claim 11.

## Patentansprüche

1. Verfahren zur Behandlung kultivierter Zellen, das die folgenden Schritte umfasst:
a) Schaffen von planaren, zweidimensionalen Objekten (Flocken), wobei diese Objekte ein Material umfassen, das bei Anwendung eines extrinsischen Reizes von dem planaren Zustand in einen gerollten Zustand übergeht,
b) Schaffen von Zellen auf den genannten Flocken, und
c) Bewerkstelligen des Übergangs der Flocken von dem planaren Zustand in einen gerollten Zustand durch Anwendung des genannten extrinsischen Reizes.

2. Verfahren nach Anspruch 1, wobei Schritt b) die folgenden untergeordneten Schritte umfasst:
b1) Aussäen von Zellen auf den genannten Flocken, und
b2) Wachsen der genannten Zellen.

3. Verfahren nach einem der vorhergehenden Ansprüche, wobei der genannte Reiz ausgewählt wird aus der Gruppe bestehend aus induzierter Änderung des pH-Wertes, induzierter Änderung der Temperatur, induzierter Exposition an elektromagnetische Wellen, induzierter Exposition an bestimmte Salze oder organische Verbindungen, oder an eine bestimmte Konzentration hiervon, Anlegen eines elektrischen Feldes, Anlegen eines magnetischen Feldes, Anwenden von Schall, Anwenden von Vibrationen.

4. Verfahren nach einem der vorhergehenden Ansprüche, wobei die Flocken ein aus einem Hydrogel bestehendes Material umfassen.

5. Verfahren nach einem der vorhergehenden Ansprüche, wobei die Flocken eine zweischichtige Struktur, eine dreischichtige Struktur, eine Mehrschicht-Struktur und/oder eine Gradientenstruktur umfassen.

6. Verfahren nach einem der vorhergehenden Ansprüche, wobei nach dem Übergang der Flocken von dem planaren Zustand in den gerollten Zustand die gerollten Flocken in einem vorgegebenen räumlichen Muster verteilt werden, um ein dreidimensionales Gewebe herzustellen.

7. Verfahren nach einem der vorhergehenden Ansprüche, wobei nach dem Übergang der Flocken von dem planaren Zustand in den gerollten Zustand die gerollten Flocken, welche verschiedene Zellentypen umfassen, in einem vorgegebenen räumlichen Muster verteilt werden, um ein dreidimensionales, heterogenes Gewebe herzustellen.

8. Verfahren nach einem der vorhergehenden Ansprüche, wobei nach dem Übergang der Flocken von dem planaren Zustand in den gerollten Zustand die gerollten Flocken mit ihren offenen Enden zueinander ausgerichtet werden.

9. Verfahren nach einem der vorhergehenden Ansprüche, wobei verschiedene Flocken, die gewachsene Zellen tragen, überlagert werden, bevor die Flocken von dem planaren Zustand in den gerollten Zustand gebracht werden.

10. Verfahren nach einem der vorhergehenden Ansprüche, wobei nach dem Übergang der Flocken von dem planaren Zustand in den gerollten Zustand die gerollten Flocken einem geschädigten Gewebebereich zugeführt werden.

11. Verfahren nach einem der Ansprüche 4 bis 10 zur Herstellung eines zweidimensionalen Objekts (Flocke) als Mikroträger für Zellen, wobei das Verfahren die folgenden Schritte umfasst:
a) Bereitstellen einer Form mit einem Raster, das Vertiefungen schafft, welche die Form der Flocken definieren;
b) Gießen eines Vormaterials in die Form; und
c) Aushärten des gegossenen Materials, um Flocken zu erhalten.

12. Zweidimensionales Objekt (Flocke) als Mikroträger für Zellen, wobei das Objekt durch Anwendung eines extrinsischen Reizes in einen gerollten Zustand gebracht werden kann und einen Zylinder oder einen zylindrischen Körper bildet, und mit einem Verfahren nach Anspruch 11 hergestellt wird.

## Revendications

1. Procédé pour traiter des cellules ayant fait l'objet d'une culture, comprenant les étapes de
a) la fourniture d'objets bidimensionnels plans (flocons), dans lequel ces objets comprennent un matériau qui, lors de l'application d'un stimulus extrinsèque, est transféré de l'état plan à un état enroulé,
b) la fourniture de cellules sur lesdits flocons, et
c) le transfert des flocons de l'état plan à un état enroulé par l'application dudit stimulus extrinsèque.

2. Procédé selon la revendication 1, dans lequel l'étape b) comprend les sous-étapes de
b1) l'ensemencement de cellules sur lesdits flocons, et
b2) la croissance desdites cellules.

3. Procédé selon une quelconque des revendications précédentes, dans lequel ledit stimulus est sélectionné parmi le groupe constitué du changement provoqué de pH, du changement provoqué de température, de l'exposition provoquée à des ondes électromagnétiques, de l'exposition provoquée à des sels ou composés organiques spécifiques, ou à une concentration donnée de ceux-ci, de l'application d'un champ électrique, de l'application d'un champ magnétique, de l'application de son, de l'application de vibrations.

4. Procédé selon une quelconque des revendications précédentes, dans lequel les flocons comprennent un matériau constitué d'un hydrogel.

5. Procédé selon une quelconque des revendications précédentes, dans lequel les flocons comprennent une structure bicouche, une structure tricouche, une structure multicouche et/ou une structure gradient.

6. Procédé selon une quelconque des revendications précédentes, dans lequel, après le transfert des flocons de l'état plan à l'état enroulé, les flocons enroulés sont distribués dans une configuration spatiale prédéfinie afin de produire un tissu tridimensionnel.

7. Procédé selon une quelconque des revendications précédentes, dans lequel, après le transfert des flocons de l'état plan à l'état enroulé, des flocons enroulés comprenant différents types de cellule sont distribués dans une configuration spatiale prédéfinie afin de produire un tissu hétérogène tridimensionnel.

8. Procédé selon une quelconque des revendications précédentes, dans lequel, après le transfert des flocons de l'état plan à l'état enroulé, les flocons enroulés sont alignés avec leurs extrémités ouvertes contiguës.

9. Procédé selon une quelconque des revendications précédentes, dans lequel différents flocons supportant des cellules ayant crû sont superposées avant le transfert des flocons de l'état plan à l'état enroulé.

10. Procédé selon une quelconque des revendications précédentes, dans lequel après le transfert des flocons de l'état plan à l'état enroulé, les flocons enroulés sont distribués à une zone tissulaire endommagée.

11. Procédé selon une quelconque des revendications 4 à 10 pour la fabrication d'un objet bidimensionnel (flocon) en tant que micro-support pour des cellules, comprenant les étapes de :
a) la fourniture d'un moule comprenant une grille qui crée des cavités définissant la forme des flocons ;
b) le coulage d'un matériau précurseur dans le moule ; et
c) le durcissement du matériau coulé pour obtenir des flocons.

12. Objet bidimensionnel (flocon) en tant que micro-support pour des cellules, l'objet étant transférable à un état enroulé par application d'un stimulus extrinsèque formant un cylindre ou un corps cylindrique et réalisé avec un procédé selon la revendication 11.
